# EUROPÄISCHE PATENTSCHRIFT

(11) **EP 0 777 706 B1**
(45) Veröffentlichungstag und Bekanntmachung des Hinweises auf die Patenterteilung: **04.03.1998**
(21) Anmeldenummer: 95930459.3
(22) Anmeldetag: 16.08.1995
(51) Int. Cl.: C09C 1/24, C09D 17/00, C01G 49/06, C01G 49/08, A61B 5/055

(54) **HOCHREINE FERROMAGNETISCHE EISENOXIDPIGMENTE**
HIGH PURITY FERROMAGNETIC IRON OXIDE PIGMENTS
PIGMENTS TRES PURS A BASE D'OXYDES DE FER FERROMAGNETIQUES

(30) Priorität: 26.08.1994 DE 4430285
(43) Veröffentlichungstag der Anmeldung: 11.06.1997
(73) Patentinhaber: BASF AKTIENGESELLSCHAFT, 67056 Ludwigshafen (DE)
(72) Erfinder: KORMANN, Claudius, D-55411 Bingen (DE); SCHWAB, Ekkehard, D-67434 Neustadt (DE); SCHLEGEL, Reinhold, D-67454 Ha loch (DE)
(86) Internationale Anmeldenummer: EP9503230
(87) Internationale Veröffentlichungsnummer: WO9606891

(56) Entgegenhaltungen:
- DATABASE WPI Week 9002 Derwent Publications Ltd., London, GB; AN 90-013781 & SU,A,1 468 905 (KELLERMAN) , 30.März 1989
- DATABASE WPI Week 8304 Derwent Publications Ltd., London, GB; AN 83-07961K & JP,A,57 200 230 (MITSUBISHI METAL) , 8.Dezember 1982
- DATABASE WPI Week 7533 Derwent Publications Ltd., London, GB; AN 75-54842W & JP,A,50 051 498 (KANTO DENKA KOGYO) , 8.Mai 1975
- DATABASE WPI Week 8249 Derwent Publications Ltd., London, GB; AN 82-05499J & JP,A,57 175 734 (SEISAN KAIHATSU KAGAKU) , 28.Oktober 1982

## Beschreibung

Die vorliegende Erfindung betrifft hochreine γ-Fe₂O₃-Pigmente und Fe₃O₄-Pigmente mit einem Teilchendurchmesser von 2 bis 100 nm, einer Sättigungsmagnetisierung über 40 nTm³/g, einer Remanenz unter 10 nTm³/g, einem Cr-Gehalt unter 40 mg/kg Pigment, einem Cu-Gehalt unter 40 mg/kg Pigment und einem C-Gehalt unter 100 mg/kg Pigment.

Außerdem betrifft die Erfindung Verfahren zur Herstellung dieser Pigmente, wäßrige Suspensionen, welche diese Pigmente enthalten, sowie die Verwendung der Pigmente und Suspensionen auf medizinischem Gebiet.

Ferromagnetische Eisenoxidpigmente können bekanntermaßen auf medizinischem Gebiet, insbesondere in Form einer wäßrigen Suspension als Kontrastmittel in der Kernspintomographie eingesetzt werden.

Die Herstellung der Pigmente erfolgt bekanntermaßen durch Umsetzung einer wäßrigen Lösung von Eisensalzen mit einer Base, wobei die im Pigment gewünschte Wertigkeit des Eisens durch Verwendung von Eisen-(II)- und Eisen (III)-Salzen oder bei zu geringer Eisen-Wertigkeit durch oxidative Behandlung der Lösung, z.B. mit Luftsauerstoff, eingestellt wird. Anschließend trennt man den Niederschlag ab und wäscht und trocknet ihn wie üblich.

Die als Ausgangsverbindungen eingesetzten Eisensalze enthalten jedoch noch geringe Mengen von Salzen anderer Metalle wie Chrom, Nickel und Kupfer. Diese Verunreinigungen stören für die meisten Anwendungszwecke nicht, sind aber physiologisch nicht unbedenklich, wenn sie wie die Kontrastmittel direkt in die Blutbahn des menschlichen Körpers gelangen.

Ferner werden an für die Kernspintomographie geeignete Eisenpigmente hohe Anforderungen hinsichtlich der ferromagnetischen Empfindlichkeit gestellt, für die die Höhe der Sättigungsmagnetisierung bei niedriger Remanenz ein Maß darstellt, um dadurch die Dosis des Mittels möglichst gering zu halten. Schließlich sollen diese Kontrastmittel möglichst feinteilig sein, damit sie sich im Blut schneller wieder auflösen und keine schwer abbaubaren Ablagerungen an den Blutgefäßwänden bilden.

Kontrastmittel mit diesen Eigenschaften standen bisher noch nicht zur Verfügung, und dementsprechend lag der Erfindung die Aufgabe zugrunde, diesem Mangel abzuhelfen.

Der Erfindung lag daher die Aufgabe zugrunde, für medizinische Zwecke, insbesondere als Kontrastmittel für die Kernspintomographie geeignete ferromagnetische Eisenoxid-Pigmente mit einem deutlich verringerten Anteil an Verunreinigungen auf technisch einfache und wirtschaftliche Weise herzustellen.

Demgemäß wurden γ-Fe₂O₃-Pigmente und Fe₃O₄-Pigmente mit einem mittleren Teilchendurchmesser von 2 bis 100 nm, einer Sättigungsmagnetisierung über 40 nTm³/g, einer Remanenz unter 10 nTm³/g, einem Cr-Gehalt unter 40 mg/kg Pigment, einem Cu-Gehalt unter 40 mg/kg Pigment und einem C-Gehalt unter 100 mg/kg Pigment gefunden.

Außerdem wurde ein Verfahren zur Herstellung dieser Pigmente gefunden und die Verwendung dieser Pigmente als Kontrastmittel in der Kernspintomographie.

Zur Herstellung solcher Pigmente ist das Eisen oder ein Eisenoxid in hochreiner Form als Vorstufe einzusetzen. Derartige Vorstufen können aus Eisenverbindungen, die einer einfachen chemischen oder physikalischen Reinigung, vorzugsweise Destillation, zugänglich sind, erhalten werden. Besonders geeignet sind dazu leichtflüchtige Eisenverbindungen, insbesondere Eisencarbonyle wie Eisenpentacarbonyl.

Eisenpentacarbonyl kann in an sich bekannter Weise hergestellt und destillativ gereinigt werden, wie es beispielsweise in Ullmann's Encyclopedia of Industrial Chemistry, 5. Aufl., Bd. A14, S. 595-601, VCH Verlagsgesellschaft mbH, 1989, beschrieben ist.

Aus Eisenpentacarbonyl kann durch thermische Zersetzung bei 60 bis 300°C, vorzugsweise 150 bis 250°C, Eisen in an sich bekannter Weise erhalten werden. Für die Herstellung von Eisenoxiden aus Eisencarbonylen kommt üblicherweise die oxidative Zersetzung von Eisenpentacarbonyl mit Sauerstoff oder einem Sauerstoff enthaltenden Gas in Betracht.

Derartige Eisenoxide, die zum Teil handelsüblich sind, bestehen zu einem erheblichen Teil aus nicht-ferromagnetischen Eisenoxiden wie α-Fe₂O₃ mit einer für die Verwendung als Kontrastmittel in der Kernspintomographie unerwünschten, hohen Partikelgröße und müssen daher zu feinteiligen, ferromagnetischen Eisenoxiden wie γ-Fe₂O₃ oder Fe₃O₄ umgearbeitet werden.

Zur Herstellung derartiger γ-Fe₂O₃-Partikel löst man das Eisen oder vorteilhaft ein Eisenoxid, insbesondere ein Oxid des dreiwertigen Eisens, in einer hochreinen wäßrigen Mineralsäure, vorzugsweise Salzsäure, auf. Bei der Verwendung von Eisen oder wenn das Eisenoxid nicht oder nur teilweise in dreiwertiger Form vorliegt, arbeitet man vorzugsweise unter oxidierenden Bedingungen, insbesondere in Gegenwart von Sauerstoff oder einem Sauerstoff enthaltenden Gas. Anschließend fällt man das ferromagnetische Pigment aus, indem man die Lösung allmählich zu einer Mineralbase, vorzugsweise Natronlauge, gibt. Zur Erzielung der gewünschten Feinteiligkeit nimmt man die Fällung unter intensivem Rühren vor, wobei die Energie vorzugsweise 0,05 bis 5 kWh pro kg des Fällungsgemisches beträgt. Für die Fällung empfehlen sich Temperaturen von 5 bis 40°C, vorzugsweise 10 bis 30°C.

Nach der Fällung trennt man den Niederschlag durch Dekantieren, Filtrieren oder Zentrifugieren ab und wäscht ihn solange, bis im Eluat keine Ionen mehr nachweisbar sind. Das Pigment kann dann getrocknet oder unmittelbar in eine gebrauchsfertige wäßrige Suspension überführt werden.

Zur Herstellung von Fe₃O₄ kommt das Auflösen von Eisen oder einem Eisenoxid in einer hochreinen wäßrigen Mineralsäure, vorzugsweise Salzsäure, in Betracht. Es sollte ein molares Verhältnis von dreiwertigem zu zweiwertigem Eisen von 2:1 oder annähernd 2:1 eingestellt werden, wenn das eingesetzte Eisenoxid dieses Verhältnis nicht oder nicht annähernd aufweist. Dies kann bei zu niedriger durchschnittlicher Eisenwertigkeit durch Oxidation, vorzugsweise mit Sauerstoff oder einem Sauerstoff enthaltenden Gas, geschehen, bei zu hoher durchschnittlicher Eisenwertigkeit durch Reduktion oder vorteilhaft durch Zugabe einer Eisen-(II)-Salz-Lösung, erhältlich z.B. durch Auflösen von reinem Eisen in einer hochreinen wäßrigen Mineralsäure unter nicht-oxidierenden Bedingungen, wie unter Schutzgas. Die weitere Umsetzung mit einer Mineralbase sollte vorteilhaft wie oben beschrieben erfolgen.

Die erfindungsgemäßen Pigmente dienen in erster Linie zur Herstellung von wäßrigen Suspensionen, die ihrerseits vor allem als Kontrastmittel für die Kernspintomographie Verwendung finden. Die Pigmentkonzentration in den Suspensionen beträgt vorzugsweise 0,0001 bis 0,6, besonders 0,001 bis 0,3 Gew.-%. Als medizinische Präparate können diese Suspensionen Hilfsmittel in den hierfür üblichen Mengen enthalten, z.B. physiologisch unbedenkliche Schutzkolloide oder Dispergiermittel wie Polyacrylate oder Proteine sowie Puffersubstanzen zur Einstellung eines pH-Wertes, der vorzugsweise 6 bis 12, besonders 6,5 bis 8 beträgt. Im übrigen wendet man die Suspensionen für die Kernspintomographie auf gleiche Weise an wie die herkömmlichen Präparate dieser Art.

### Beispiel

Zu einer Suspension von 2 kg hochreinem α-Fe₂O₃ in 10 kg destilliertem Wasser wurden 7,8 kg 38 gew.-%-iger hochreiner Salzsäure gegeben und bis zur Lösung des Eisenoxids erhitzt.

Nach dem Abkühlen auf Raumtemperatur wurde diese Lösung mit 4,7 kg einer 33 gew.-%-igen Eisen-(II)-chloridlösung vermischt, die durch Auflösen von Reinsteisen in hochreiner Salzsäure hergestellt worden war.

Die resultierende Lösung wurde dann unter Stickstoff allmählich innerhalb von 13,5 h bei 20°C unter intensivem Rühren (0,9 kWh Rührenergie) zu 17,5 kg einer 25 gew.-%-igen NaOH-Lösung gegeben.

Der Niederschlag wurde abfiltriert, gewaschen und getrocknet.

Er enthielt als Verunreinigungen pro kg Pigment 24 mg Chrom, <30 mg Kupfer und 80 mg Kohlenstoff und hatte einen mittleren Teichendurchmesser von 15 nm.

Die Sättigungsmagnetisierung betrug 74 nTm³/g und die Remanenz 5 nTm³/g.

## Patentansprüche

1. γ-Fe₂O₃-Pigmente und Fe₃O₄-Pigmente mit einem mittleren durchschnittlichen Teilchendurchmesser von 2 bis 100 nm, einer Sättigungsmagnetisierung über 40 nTm³/g, einer Remanenz unter 10 nTm³/g, einem Cr-Gehalt unter 40 mg/kg Pigment, einem Cu-Gehalt unter 40 mg/kg Pigment und einem C-Gehalt unter 100 mg/kg Pigment.

2. Wäßrige Suspensionen, enthalt-end ein Pigment gemäß Anspruch 1.

3. Verfahren zur Herstellung von γ-Fe₂O₃-Pigmenten gemäß Anspruch 1, dadurch gekennzeichnet, daß man Eisen oder ein Eisenoxid, welches unter 40 ppm Cr, unter 40 ppm Cu und unter 100 ppm C enthält, in einer hochreinen wäßrigen Mineralsäure auflöst und aus dieser Lösung das γ-Fe₂O₃ bei 5 bis 40 °C unter intensivem Rühren mit einer Mineralbase ausfällt, wobei man im Falle des Eisens, oder wenn das eingesetzte Eisenoxid nicht oder nur teilweise in dreiwertiger Form vorliegt, unter oxidierenden Bedingungen arbeitet, und den so erhaltenen feinteiligen Niederschlag wäscht und trocknet.

4. Verfahren zur Herstellung von Fe₃O₄-Pigmenten gemäß Anspruch 1, dadurch gekennzeichnet, daß man Eisen oder ein Eisenoxid, welches unter 40 ppm Cr, unter 40 ppm Cu und unter 100 ppm C enthält, in einer hochreinen wäßrigen Mineralsäure auflöst, ein molares Verhältnis von dreiwertigem zu zweiwertigem Eisen von 2:1 oder annähernd 2:1 einstellt, wenn die Lösung dieses Verhältnis nicht oder nicht annähernd aufweist, und aus dieser Lösung das Fe₃O₄ bei 5 bis 40 °C unter intensivem Rühren mit einer Mineralbase ausfällt und den so erhaltenen feinteiligen Niederschlag wäscht und trocknet.

5. γ-Fe₂O₃-Pigmente und Fe₃O₄-Pigmente, erhältlich nach den Verfahren gemäß Anspruch 3 bzw. Anspruch 4.

6. γ-Fe₂O₃-Pigmente und Fe₃O₄-Pigmente gemäß Anspruch 1 sowie wäßrige Suspensionen dieser Pigmente gemäß Anspruch 2 zur Verwendung auf medizinischem Gebiet.

7. Verwendung der Suspensionen gemäß Anspruch 2 zur Herstellung von Kontrastmitteln für die Kernspintomographie.

## Claims

1. A γ-Fe₂O₃ pigment or Fe304 pigment with an average particle diameter of 2-100 nm, a saturation magnetization above 40 nTm³/g, a remanence below 10 nTm³/g, a Cr content below 40 mg/ kg of pigment, a Cu content below 40 mg/kg of pigment and a C content below 100 mg/kg of pigment.

2. An aqueous suspension containing a pigment as claimed in claim 1.

3. A process for preparing γ-Fe₂O₃ pigments as claimed in claim 1, which comprises dissolving iron or an iron oxide which contains below 40 ppm Cr, below 40 ppm Cu and below 100 ppm C in a high-purity aqueous mineral acid, and precipitating γ-Fe₂O₃ from this solution at 5-40°C with vigorous stirring using a mineral base, operating under oxidizing conditions in the case of iron or if the iron oxide employed is not present in trivalent form or is only partially present in trivalent form, and washing and drying the fine-particle precipitate obtained in this way.

4. A process for preparing Fe₃O₄ pigments as claimed in claim 1, which comprises dissolving iron or an iron oxide which contains below 40 ppm Cr, below 40 ppm Cu and below 100 ppm C in a high-purity aqueous mineral acid, adjusting to a molar ratio of trivalent to divalent iron of 2:1 or approximately 2:1 if the solution does not (approximately) have this ratio, and precipitating Fe304 from this solution at 5-40°C with vigorous stirring using a mineral base, and washing and drying the fine-particle precipitate obtained in this way.

5. A γ-Fe₂O₃ pigment or Fe₃O₄ pigment obtainable by the process as claimed in claim 3 and claim 4 respectively.

6. A γ-Fe₂O₃ pigment or Fe₃O₄ pigment as claimed in claim 1, and aqueous suspensions of these pigments as claimed in claim 2, for use in the medical sector.

7. The use of the suspensions as claimed in claim 2 for the preparation of contrast agents for magnetic resonance imaging.

## Revendications

1. Pigments de γ-Fe₂O₃ et pigments de Fe₃O₄ à un diamètre de particule moyen de 2 à 100 nm, une magnétisation de saturation supérieure à 40 nTm³/g, une rémanence inférieure à 10 nTm³/g, une teneur en Cr inférieure à 40 mg/kg du pigment, une teneur en Cu inférieure à 40 mg/kg de pigment et une teneur en C inférieure à 100 mg/kg de pigment.

2. Suspensions aqueuses contenant un pigment selon la revendication 1.

3. Procédé de préparation des pigments de γ-Fe₂O₃ selon la revendication 1, caractérisé par le fait que l'on dissout du fer ou un oxyde de fer contenant moins de 40 ppm de Cr, moins de 40 ppm de Cu et moins de 100 ppm de C dans un acide minéral aqueux à haute pureté et à partir de cette solution, on précipite le γ-Fe₂O₃ à des températures de 5 à 40°C sous agitation intensive à l'aide d'une base minérale, sous réserve que dans le cas du fer ou lorsque l'oxyde de fer mis en oeuvre n'est pas à l'état trivalent ou n'est qu'en partie à l'état trivalent, on opère en milieu oxydant, après quoi on lave le précipité en fines particules ainsi obtenu et on le sèche.

4. Procédé de préparation des pigments de Fe₃O₄ selon la revendication 1, caractérisé par le fait que l'on dissout le fer ou un oxyde de fer contenant moins de 40 ppm de Cr, moins de 40 ppm de Cu et moins de 100 ppm de C dans un acide minéral aqueux à haute pureté, on règle le rapport molaire fer trivalent/fer divalent à 2 : 1 ou à peu près à 2 : 1 lorsque la solution n'est pas à ce rapport ou au voisinage de ce rapport, et à partir de cette solution, on précipite le Fe₃O₄ à des températures de 5 à 40°C sous agitation intensive à l'aide d'une base minérale, puis on lave le précipité en fines particules ainsi obtenu et on le sèche.

5. Pigments de γ-Fe₂O₃ et pigments de Fe₃O₄ obtenus par les procédés respectifs selon la revendication 3 et revendication 4.

6. Pigments de γ-Fe₂O₃ et pigments de Fe₃O₄ selon la revendication 1 et suspensions aqueuses de ces pigments selon la revendication 2, pour l'utilisation dans le domaine médicale.

7. Utilisation des suspensions selon la revendication 2, pour la préparation d'agents de contraste pour la scannotomographie nucléaire.
